# EUROPEAN PATENT SPECIFICATION

(11) **EP 2 056 969 B1**
(45) Date of publication and mention of the grant of the patent: **03.10.2018**
(21) Application number: 07800572.5
(22) Date of filing: 31.08.2007
(51) Int. Cl.: B05B 15/00, A61L 9/14, B05B 12/00, B05B 17/00

(54) **APPARATUS AND METHODS FOR ELIMINATING AND PREVENTING VEHICLE ODORS**
VORRICHTUNG UND VERFAHREN ZUR BESEITIGUNG UND VERHINDERUNG VON FAHRZEUGGERÜCHEN
DISPOSITIF ET PROCÉDÉS POUR LA SUPPRESSION ET LA PRÉVENTION DES ODEURS DANS UN VÉHICULE

(30) Priority: 01.09.2006 US 824370 P
(43) Date of publication of application: 13.05.2009
(73) Proprietor: CPS Products Canada Ltd., Niagara Falls, ON L2E 6S5 (CA)
(72) Inventor: ALDANA, Leonardo, Mississauga, Ontario L4Y 1M5 (CA); CHOCHOL, Andrew, Stanislaw, Alliston, Ontario L9R 1A8 (CA); FERRARO, Tony, Mississauga, Ontario L5B 1W2 (CA)
(74) Representative: Becker Kurig Straus
(86) International application number: PCT/CA2007/001548
(87) International publication number: WO 2008/025168

(56) References cited:
- US-A- 3 255 626
- US-A- 5 657 926
- US-A- 5 657 926
- US-B1- 6 712 287
- Airocomatic Product Sheet (WYNN) 2002
- Aircomatic II Product Sheet (WYNN) 2005
- Airco-Clean Product Information Sheet (WYNN) March 2006

## Description

### BACKGROUND

Vehicles often accumulate odors inside their cabins during their lifetime of use. Such odors can be caused in a variety of ways and by a variety of sources. For example, objects left inside the vehicle, volatile organic compounds (VOCs) from the cabin interior materials, activities such as smoking and eating and the accumulation of dust and other pollutants suspended in the air can all contribute to the accumulation of odors. Eventually odors inside a vehicle become annoying and in some cases they may become a health risk if the source of odor involves bacteria, fungi or other microorganisms.

Besides the obvious surfaces that become contaminated with pollutants, like the seats, dashboards, carpets and other visible parts of the interior of a vehicle there are some hidden areas that create a perfect environment for pollutants, such as fungi, to accumulate and grow to a level where they can be noticed by smell even before they are visible. There are also many germs, bacteria and mold that are present and may be never cause an odor.

One location for the growth and/or accumulation of hidden pollutants is in the interior of the air conditioning system. Typical air conditioning units include a chamber, where the refrigerant serpentine, also known as evaporator core, is embedded. Under normal operating conditions of a properly functioning air conditioning system, the serpentine condenses the moisture coming into the chamber due to the interaction between temperature, the existing dew point, and the relative humidity inside and outside the vehicle. In this process, the air entering the system contacts the cold interior parts of the system which retain and condense the humidity from the air. The cooler drier air comforts passengers once it exits the system, vents, and enters the vehicle cabin.

The condensation causes water to flow along the walls of the serpentine and the evaporator as well as the inside of the ventilation system. The accumulated water exits the chamber through a drain hole/hose designed specifically for this purpose. However, the surfaces inside the unit and ventilation system can remain humid for extended periods of time that vary from minutes to months depending on usage and climate conditions.

Year after year the air conditioning system is turned on and off and suspended dust, dirt, pollen, fungus, bacteria and other polluting agents in the air enter the chamber passing into the evaporator chamber as the air conditioner blower draws air through the system both from the cabin and from the exterior of the vehicle through the air intake. Some of the particles from the polluted air adhere to the moist surfaces of the serpentine or other internal walls of the chamber as the air passes through the evaporator. In addition, some of the pollutants pass through the system and can become deposited over the interior surfaces of the cabin. The accumulated particles on the moist surfaces of the evaporator provide an environment in which microbes can grow, particularly in the absence of UV light from the sun. The growth of microbial pollutants inside the evaporator further increases the amount of pollutants and odors that can enter the cabin in the airflow created by the blower. Automobile manufacturers have recognized a need for cleaning air conditioning systems for years. For example, US 5,385,028 to General Motors discloses what is said to be a method of eliminating odor in a heat pump system of a vehicle that includes the steps of detecting removal of the vehicle passengers and ignition key after use of the cooling mode or air conditioning of the passenger compartment heat exchanger, operating the blower, reversing the flow of refrigerant in the heat pump to place the passenger compartment heat exchanger in heating mode to remove latent moisture. US 5,259,813 to Mercedes discloses a method for deciding whether to recirculate air. The quality of the external air is determined by means of a pollutant sensor. The quality of the internal air is determined by calculation taking account of the air quantities introduced from outside into the internal space. A decision between air supply operation and air recirculation operation is then made on the basis of a comparison of the air qualities inside and outside. The pollutant sensor is preferably located in a casing whose internal space is accessible to gases through an opening which is preferably sealed by a gas-permeable membrane to eliminate odor. The proliferation of carbon air filters in new vehicles provides an indication of the consumers awareness of the air space in a vehicle and their desire for cleaner air. Even the US military has needs for air quality of cooled air as evidenced by US Patent No. 5,386,823. US 5,657,926 to Toda discloses an ultrasonic atomizing device capable of continuous and stable liquid atomization under the resonance frequency variation associated with temperature change. The device comprises an ultrasonic transducer, a low direct current voltage power supply, and a chamber.

Contaminants and odors can also originate from inside the passenger compartment and these also can circulate through the ventilation system and inside the evaporator when the air conditioner is operating. As an example, tobacco smoke originating from within the passenger compartment can cause odor in fabric headliners, upholstery and carpets all of which can be transported through the ventilation system and inside the evaporator. Further, any moisture accumulations in any part of the cabin can provide a place for mold and bacteria to grow. The mold can generate spores that can become suspended in the air inside the cabin. These spores can then re-circulate through the air conditioning system. Because of the moisture and temperature activity inside the evaporator and the lack of light, many of these contaminants and particles tend to accumulate inside the evaporator unit, creating layers of what appears to be "mud". When the air conditioning is turned on spores in the system can be blown out into the cabin which can actually create a health risk in certain individuals. At best, this situation creates an annoying bad-smelling odor every time the A/C and/or the heater are turned on.

Methods for removing or treating these mold, bacteria and odors inside the evaporator and ventilation system have been developed. One method is to spray a foaming aerosol solution through the evaporator drain hole. The foam then expands into foam inside the evaporator. However, the rapid expansion from an aerosol to a foam state prevents the foam from effectively reaching the upper recesses of the evaporator In addition, the method is complicated by the need to either remove the evaporator or raise the vehicle on a lift in order to reach the drain hole, or drill a hole in the evaporator case to allow a straw type aerosol injector access. These are all labor intensive operations requiring a person to position the vehicle appropriately, position and hold the aerosol can while depressing the valve releasing its contents. Airsept, Inc. provides one such product. Alternatively, electronics can be used to keep the evaporator dry. *See e.g.,* US Patents 5,899,082 and 6,840,051.

Another method involves spraying a non-foaming aerosol solution into the exterior-located air intake, while the blower motor is running. However, because the atomized particles of the spray are heavier than air, they do not travel effectively and far enough to reach the inside of the evaporator or the entire ventilation system. Neither of these solutions is designed to treat interior odor-causing contaminants and both are labor-intensive in that the operator must continuously depress the valve on the aerosol can in order to release it's contents.

Other methods for treating odor-causing contaminants involve generating a mist out of a cleaning solution inside the passenger compartment. Spray devices for carrying out this process can be pointed into the intake(s) of the air conditioning re-circulating system of the vehicle while the air conditioning system is in operation. The mist-saturated air then circulates through the evaporator and the ventilation system, the cleaning solution can condense on the inside walls of the evaporator and vents and can flow into the passenger compartment. As this happens, the cleaning solution comes into contact and interacts with contaminants, thus removing or reducing mold, bacteria and odors.

Several methods are known for vaporizing a cleaning solution. In one method a vapor is created by using ultrasonic piezo-transducer. However, such devices have a number of drawbacks in the automobile cleaning environment such that they have not been used in the past. For example, existing devices require a high voltage alternating current (AC) to operate, which places limitations on the application of these devices, as they are dependent on this type of power being available in close proximity in order for a vehicle to be serviced with this device and method. AC-powered devices of this nature are limited to areas of a building or shop with access to AC electricity. Ultimately, the current required to produce a sufficient amount of vapor from such a device has led manufacturers away from making portable systems.

Several devices operated by high voltage alternating current are known. For example, Wynn's AIRCOMATIC® Ultrasonic Air Conditioning Cleaning System. Wynn's AIRCOMATIC II® Ultrasonic Heating, Ventilation & Air Conditioning Cleaning System, which uses ultrasonic technology but needs to be connected to high AC voltage. The VAPORTEK® Restorator uses electric heat, but no ultrasonic technology and is powered by either AC or low voltage direct current (LDVC) in different versions. The AIRCLEAN-EVAPORATOR® which uses same technology as the VAPORTEK® Restorator, but is only powered by a high AC voltage version and the Wurth EVAPOclean®, which uses ultrasonic technology but requires high voltage AC power supply.

Each of these devices requires an electrical cable, or extension cord, that extends from an AC power source to the unit which is positioned inside a vehicle for use. The cable or cord transits through the vehicle's window or door. The vehicle window or door must be shut tight against the cord in order to minimize external air contaminants from entering the vehicle and not allowing the treatment to exit the vehicle. However, the thickness of the cable or cord leaves a gap to external air in the vehicle compartment which decreases the effectiveness of the service as a portion of the mist escapes through the gap. Moreover, the resulting pressure exerted from the window or door on the cable or cord can be sufficient to cut or strip insulation material and expose live wires that can lead to a short circuit and potentially a fire since these devices work with High AC Voltage. For these reasons such devices have not come into popular use.

For a piezoelectric transducer to operate properly, it is important that the liquid that is converted to mist will not damage the device by corrosion, scaling or accumulation of residues. Since the device is meant to be used by a diverse group of people, there is the potential that different cleaning solutions may be used, either accidentally or deliberately, in the device. All of the known products allow for any type cleaning solution or chemical to be used, providing no control over what is used in the unit. This makes it highly likely that eventually the unit will be damaged by an unsuitable cleaning solution or be made unsafe to the occupants by someone using a commercially available cleaning solution like Windex, Febreeze, bleach or chlorine. The refill openings of known devices are even big enough to drop solids into the machine that will affect its performance. This is yet another reason why such devices are not widely used for cleaning automobile ventilation systems.

Some of the existing devices are configured such that a fan or blower cools down the electric circuit that drives the piezoelectric transducers. The circuit board is located in a chamber away from the "misting" chamber, but is still connected to it through one or more ducts. This arrangement makes it possible that given certain inclination, tilting or movements of the device, some fluid may travel to the circuit board's chamber and come in contact with the electric components. This creates a risk of device malfunction and also of a short circuit or shock. Another consequence of this arrangement is that the cooling air must travel from the fan/blower through the circuit board through the duct and then through the "misting" chamber. The resistance this path creates to the air flow, forces the fan/blower to be over powered to ensure that enough air flows to cool the circuit board and still generates a sufficient volume of mist for the cleaning treatment cycle.

Some of the existing devices are configured in such a way that the turbulence, foam and/or splashing created by the piezoelectric transducers inside the "misting" chamber soaks some special mechanisms or components in the fluid, affecting their performance and durability of the device. Some devices have a level measuring system that signal the machine to stop when the fluid is low The splashing and turbulence in these devices can in some instances create false signals stopping the device prematurely. In some cases the foaming and turbulence can also affect the amount of mist coming out of the device.

The misting performance of a piezoelectric transducer is dependent on the amount of fluid over it and this is influenced by the angle of tilt of the device. Since existing devices don't have any way to orient the user on whether the equipment is really in an upright position, the performance of the equipment may be affected negatively without the knowledge of the user. For example, when the machine includes a level sensor and the machine is tilted a number of degrees from the horizontal, the machine may either work longer, which would damage the piezoelectric transducers if the level goes too low on one side; or the machine may stop its cycle earlier, which causes an insufficient amount of fluid use in the treatment.

The existing devices require close monitoring to ensure that treatments are finished and that machines are working properly. Most of the known devices display such information on their sides, which means that to monitor them in an automobile, the door of the vehicle must be opened and the technician must lean over and check the side of the device for that information.

For all of the above reasons known devices have not come into widespread use and new devices and methods for cleaning automobile ventilation systems are needed.

### SUMMARY

A mist-generator according to claim 1 is provided. The device can operate from a vehicle's direct current power supply, such as a 12 or 24 volt direct current electric power supply. Because the device operates from Low Voltage Direct Current (LVDC) it can be used in a wide variety of vehicles. The device can also be operated through special transformers or power supplies. Another advantage of this device is that it can be operated in virtually any location including such remote areas as parking lots, farms, trade shows, on-the-road demonstrations, and the like. Further aspects and embodiments are disclosed in die dependent claims.

Additional features and advantages are described herein, and will be apparent from, the following Detailed Description and the figures.

### BRIEF DESCRIPTION OF THE FIGURES

Figure 1 provides an elevated view of an embodiment of the device of the present invention.

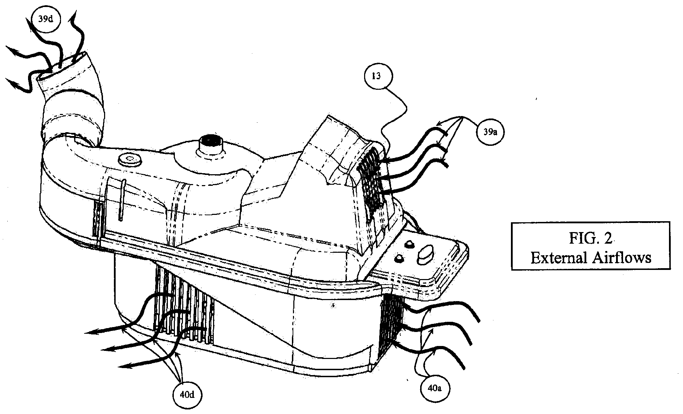

Figure 2 provides an elevated view of an embodiment of the device and illustrates one pattern of external air flows.

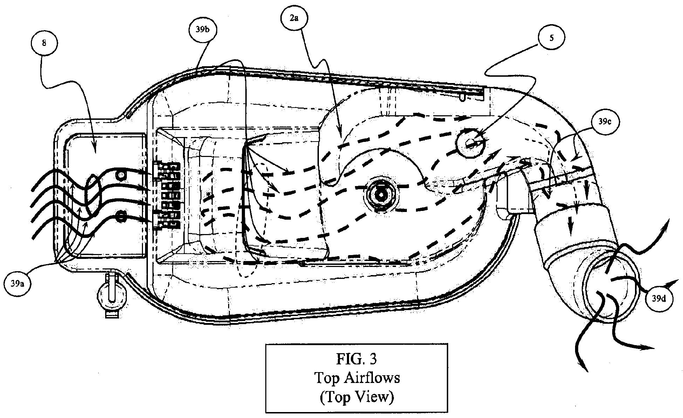

Figure 3 provides a top view of an embodiment of the device and illustrates one pattern of air flow through the top of the device.

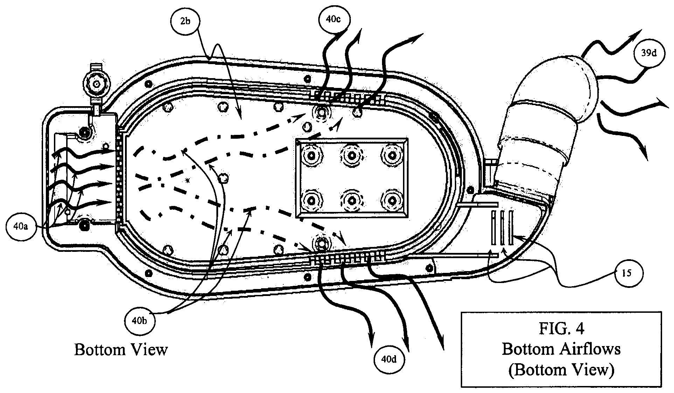

Figure 4 provides a bottom view of an embodiment of the device and illustrates air flow through the bottom of the device.
Figure 5 provides a top view of the top cover of the device having splash guards.
Figure 6 provides an illustration of a cross section of an embodiment of the device in which the trap is closed during normal operation.
Figure 7 provides an illustration of a cross section of an embodiment of the device in which the trap is closed during normal operation.
Figure 8 provides an illustration of a cross section of an embodiment of the device having a trap and helix in apposition showing abnormal flow as a result of an attempt to fill the device through the outlet nozzle.
Figure 9 provides an illustration of a cross-section of one embodiment of the device filling mechanism.
Figure 10 provides an illustration of a cross section of one embodiment of the device illustrating both a fluid flow and an air flow.
Figure 11 provides an illustration of an anti-splash guard and an anti-foam guard.

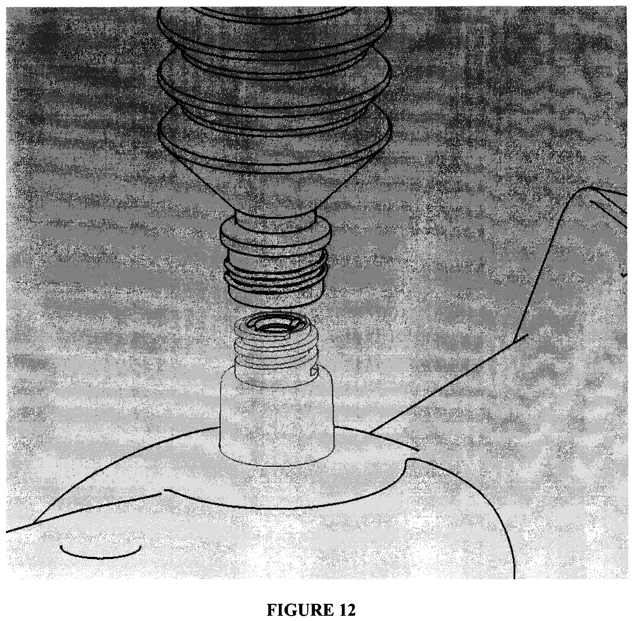

Figure 12 provides an elevated perspective view of a refill container in close proximity to an inlet.

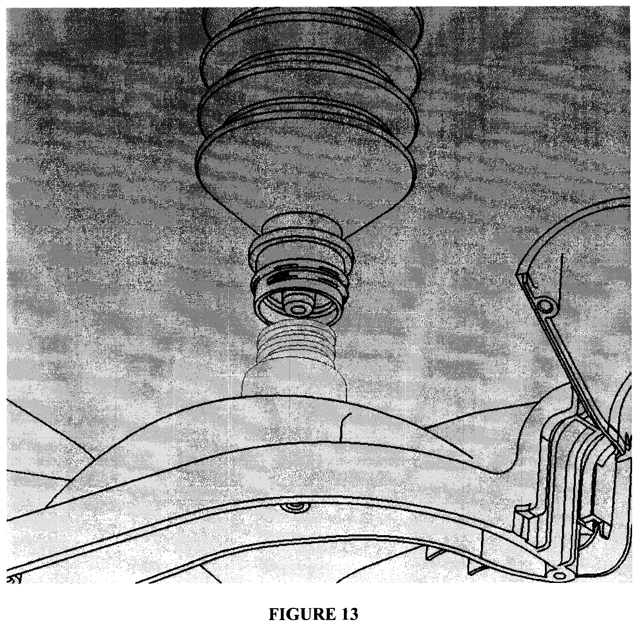

Figure 13 provides a perspective view of a refill container in close proximity to an inlet from beneath the inlet.

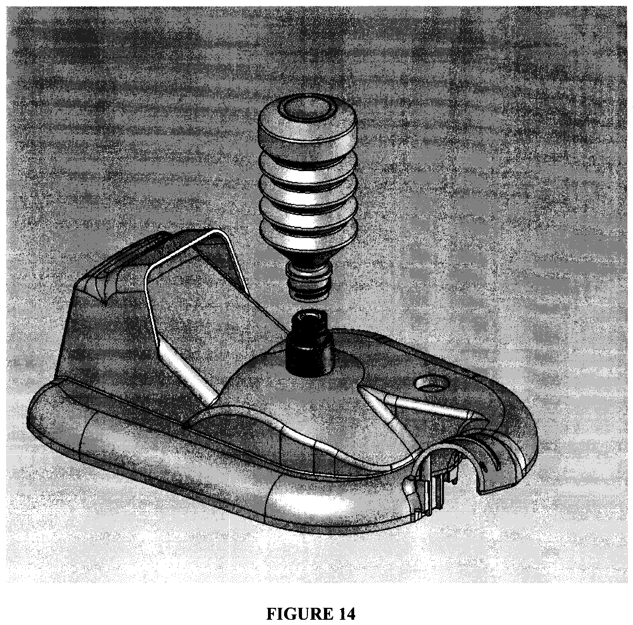

Figure 14 provides an elevated perspective view of a refill container in close proximity to an inlet.

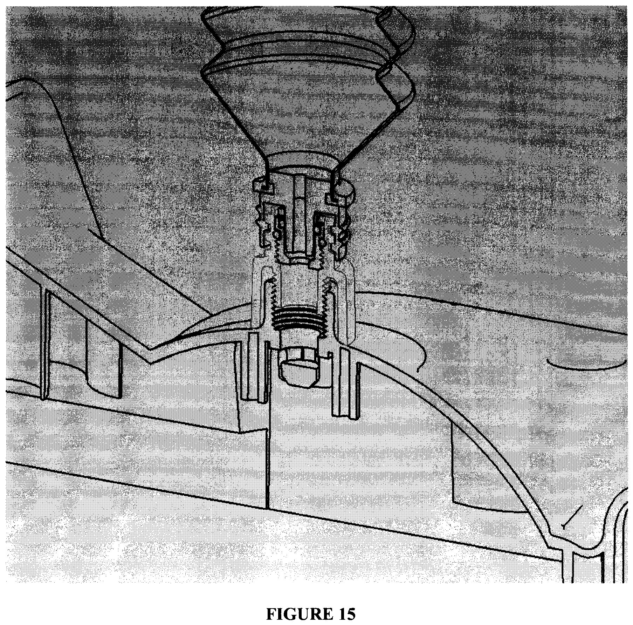

Figure 15 provides a cross section view of a refill container in close proximity to an inlet.

### DETAILED DESCRIPTION

For purposes of the present application the phrase "mist-generating" refers to the conversion of a liquid into very small droplets, which are expelled from the unit looking as fog, vapor, fume, fine spray, or having other visual similarities.

The abbreviation "AC" refers to alternating current which is normally above 90 volts and which is typically available from electrical wall outlets and in some cases from special power supply stations.

The abbreviation "A/C" refers to Air Conditioning.

The abbreviation "LVDC" refers to low voltage direct current as provided by a vehicle power supply.

The abbreviation "VOCs" refers to volatile organic compounds, such as aldehydes, ketones, hydrocarbons and the like. They can be expelled by some substances and materials, which in some cases raises health concerns. VOCs are often the cause of "new car smell" as new materials, due to their chemical process of manufacture and/or finish continue expelling these odors even after months of reaching the end-customers.

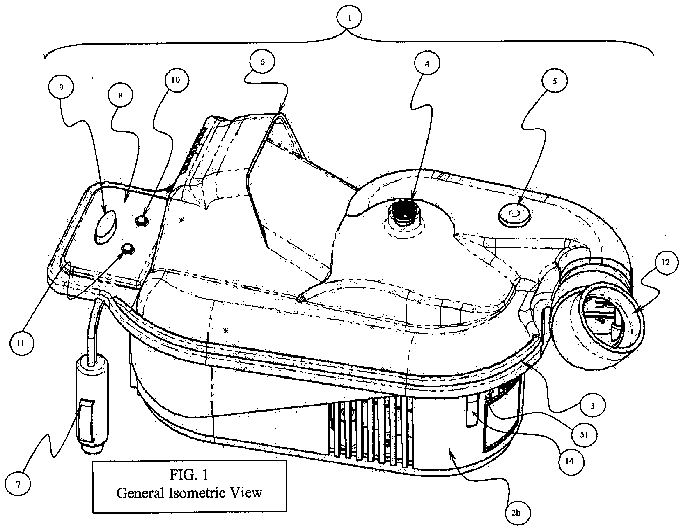

As illustrated in Fig. 1 a mist-generator 1 is disclosed that is particularly useful for automotive mold, bacteria and odor treatment and removal. The device can operate from a vehicle's 12 or 24 volt direct current electric power supply through a power adapter 7. Because the disclosed mist-generating device 1 can be powered by a LVDC power supply, the risk of shock is greatly reduced as compared to devices that are powered by AC. This is particularly significant because the fluid used for the cleaning is often an electro conductive liquid composition.

In this embodiment no wires leading from the vehicle are needed because a LVDC power source is typically available inside the cabin of most vehicles. The point of electrical connection inside the vehicle cabin can be a cigarette lighter or similar electrical outlet. In this configuration the risk of a power cord being pinched, shorted circuited or cut is completely avoided as are gaps in doors or windows that are necessary when known devices are used. Thus, undesired leaks of the cleaning solution's mist are also avoided.

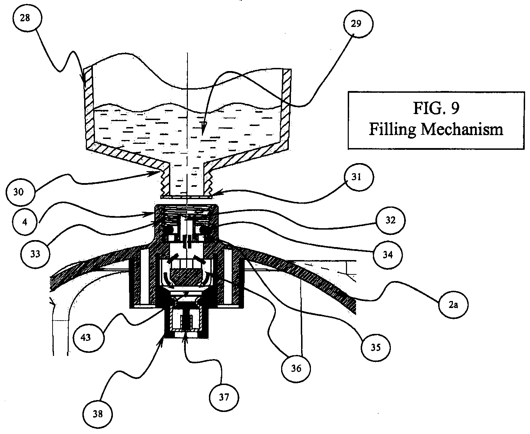

In an embodiment, a mechanism can be provided in the sprayer that prevents the introduction of undesired objects or unknown chemicals into the device. To this end, the device 1 can be configured with a unique filling mechanism, shown in more detail in Fig. 9, that allows only specific refill containers 28 to be attached to the device; containers specifically designed for use with the device 1. An external locking mechanism, such as threading can be located on a neck 30 that makes up the orifice. The orifice can be used to expel the cleaning fluid 29 from the container into the mist generator 1. Filled refill containers 28 can be covered by a thin material 31, such as a foil, during transportation and storage prior to their use such that the cleaning solution is held in place in the tube and is not contaminated.

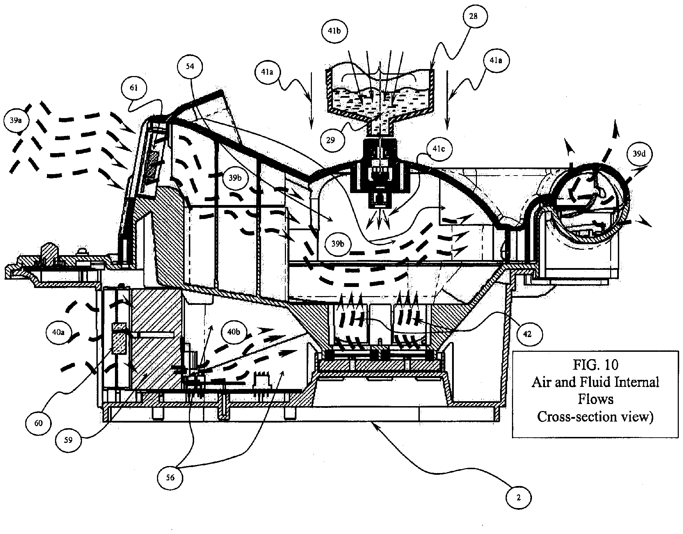

The spray device 1 can have an inlet 4 adapted to receive the locking mechanism, such as the threaded neck 30 of the refill container 28. Thus, the inlet 4 could be threaded such that a threaded refill container 28 could be screwed into the inlet 4. The inlet 4 can also be adapted with a cutter 32 that pierces the thin material 31 and allows for the fluid 29 to pass from the refill container 28 into the mist generator device 1 after refill container 28 is screwed onto the inlet nozzle 4. Preferably, cutter 32 is configured to prevent the foil from being shredded into pieces so that chips of the foil will not fall into the sprayer or clog the inlet 4 of the device 1. The cutter 32 can be mounted to inlet 4 using the same threading that the refill container 28 uses and can be screwed into the inlet 4 until it bottoms down. Cutter 32 can have a passage inside the cutting edge that allows the fluid 29 to pass through once the container 28 has been pierced. The threading on the neck 30 of the refill container 28 can be inside the neck 30 such that the neck 30 screws into an externally threaded inlet 4 on the machine 1. Under the cutter 32 and inlet 4 the device 1 can have a check valve 43 that prevents fluid entry by gravity. In such a case, positive pressure must be applied to the refill container 28 to force liquid 29 into the storage chamber 41c (Fig. 10) of the mist generator 1. For example, the refill container 28 can simply be squeezed, such as in tube type refill containers 28.

Refill container 28 can be any type of container that can hold the mist solution 29 such that it can be introduced in to the mist-generating machine 1. Refill container 28 can have any shape and be made of many materials including pliable plastics and metals. One exemplary shape is similar to a tube, such as a toothpaste tube, having an orifice on one end and closed on the other. Squeezable tubes, bottles, cans, bags can be used so long as they are adapted to lock in to the machine. Many other collapsible or flexible containers are known and can be used to refill the mist generator so long as they are adapted to include the same neck-locking design 30. Alternatively, pressure can be applied to the device using a syringe type refill container 1 or a caulking-gun concept in combination with the locking mechanism.

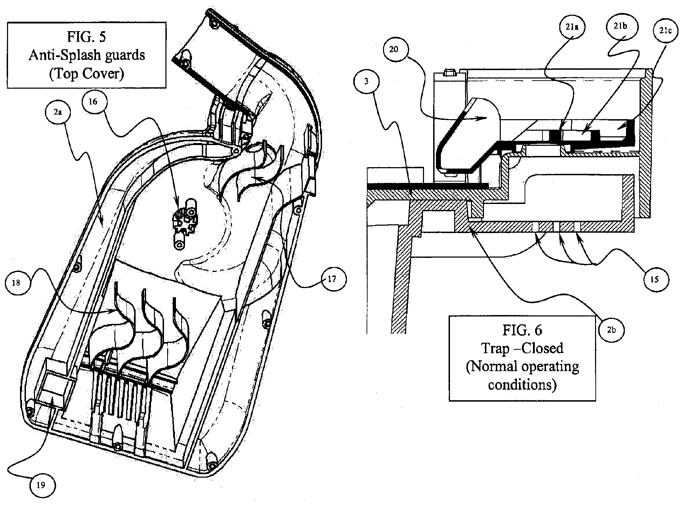

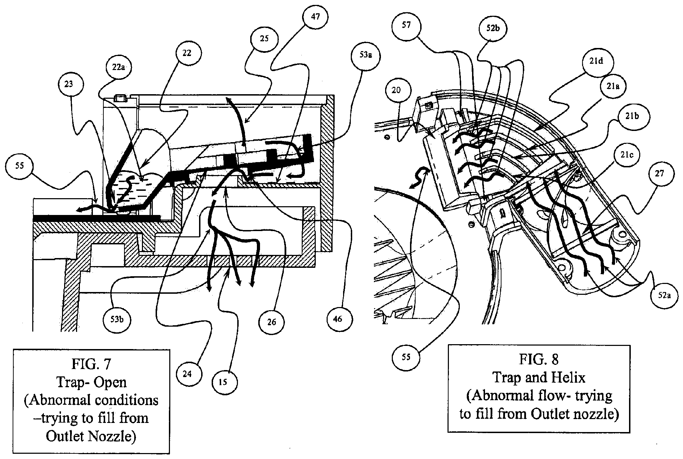

The mist generator 1 has a spray outlet 12 of sufficient size to allow enough mist to come out to cleanse an automobile ventilation system. The size of the outlet 12 opening allows for the possibility that undesirable solutions or foreign materials could gain entry into the misting chamber 41c. To avoid this, as best illustrated in Figs. 6-8, the device can be configured with a trap 20 that involves a moving piece inside the machine 1. Such a trap 20 can have a horizontal axis and two protrusions 46 that emerge from the body of the trap 20. The protrusions 46 serve as a pivoting point for the trap to flip up, as in Fig. 7 and down, as in Fig. 6. At one of the sides of the axis, there can be one or more small volume receptacles 22 with one or more holes 23 at the bottom. The other side can be flat. As best illustrated in Fig. 8, the flat part can have at its top surface one or more ridges 21a-d that direct any fluid 22a coming from the inlet over the axis and into the receptacle 22. On its bottom, the trap 20 has a ridge 24 and surface that closes an opening 26 on the bottom surface of the sprayer. As best illustrated in Figs. 6 and 7, the receptacle 22 can be designed in such a way that as fluid 22a accumulates in it will lower down as the weight of the fluid forces it down and then will retain more fluid as it lowers. In the embodiment shown in Fig. 7, the receptacle 22 stops lowering as soon as it makes contact with another part or surface of the device. When this happens, the flat part of the trap 20 rises as the trap 20 flips over its axis shafts. As the flat part rises, the opening on the device 1 is uncovered and most of the fluid being poured spills out of the device 1. As long as the fluid flow keeps sufficient pressure against the bottom surface of the trap fluid will flow through the device's opening 26. After the fluid flow stops, the receptacle 22 of the trap 20 loses weight as the fluid 22a goes through the small orifice and the trap 20 tilts back to its original horizontal position to seal the opening 26 of the device.

In an embodiment best shown in Fig. 8, a helix shaped piece 27 can be included in the spray nozzle 12 such that air and mist can come freely out of the spray nozzle 12 but the entry of objects into the device such as those that could be used to maintain the trap 20 closed, is precluded.

The spray device 1 can include more than one blower 60 and 61 in Fig. 10. Separate blowers 60 and 61 can be positioned in separate chambers. In one chamber one or more blowers 60 and 61 can be used to push air through the electronics to keep them cool, and in a separate chamber 41c where the mist is produced, a separate set of fans can be used to spray the mist out of the device 1 through the outlet 12. By maintaining separate chambers the risk of having the fluid contacting the electronics is reduced and there is a lower probability of a short circuit or shock.

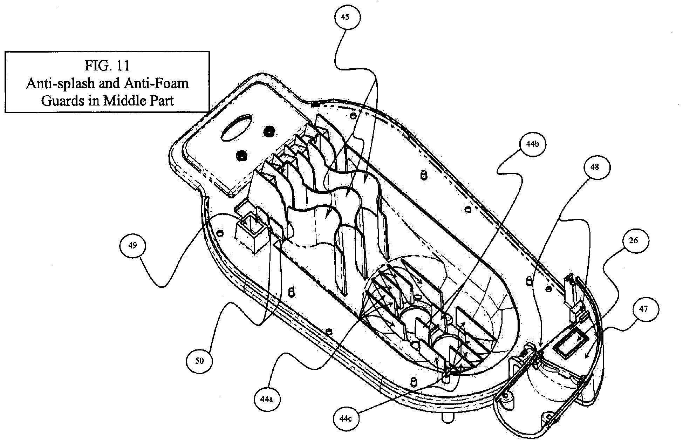

In an embodiment best illustrated in Fig. 11, the spray device 1 can have an internal structure in the misting chamber 41c that reduces turbulence and splashing. Such a mechanism helps maintain performance of the delicate components and mechanisms in the chamber 41c. The anti-splashing and anti-foaming features can be a set of ribs 45 and 44a-c around the piezo transducers 42 that will act as wave-breakers and will reduce the turbulence of the fluid levels above the piezos 42, which in turn helps to maintain a consistent output of mist. The device can have a series of "S" shaped ribs 45 that allow air to pass through, but doesn't allow splashing or water displacement which normally occurs in a straight line.

The device I can include a level vial as best shown in 5 of Fig. 1 which can be positioned at the top surface to let a user know when the machine is level or within an acceptable inclination in which the machine will work correctly. This helps to prevent the user from placing the machine at an angle that may reduce the performance characteristics of the device 1. The device 1 may also have an electrical interlock to prevent the mist generator device I from operating if the angle is outside an acceptable range.

The spray device 1 can also include a mechanism for notifying an end user that the treatment is in progress, finished or when there is a problem with the machine. The mechanism can be a top display with light(s) 10 and 11, such as LEDs, which shows the status of the machine and/or the treatment without having to open the door of the car. Depending of the frequency of the flashing, the user can determine if the machine is still running or is stopped because the treatment time was complete, or whether there was a problem with the machine. The display can also be a readable display that describes the sprayer status or it can be a remote wired or wireless display showing having indicator lights or a readable display. Such a display can be placed either inside of the vehicle in a visible place or placed outside of the vehicle at a convenient location where the user can be conveniently observed. The device can be configured with a sound system such that an attached or remote buzzer can indicate the status of the machine or the stage of the treatment. A light emitting source 10 and 11 can be used that emits light beams through the mist to show the effectiveness of the treatment to the user(s).

The disclosed spray device provides an easily controllable sprayer in a reduced size, that can produce a very fine spray of cleaning solution, comparable to a mist.

A portable piezo 42 based, mist-generating device 1 is provided that can connect to a vehicle's Low Direct Current Voltage (LDCV) power supply in order to perform cleaning/odor removal services on exposed and hard-to reach surfaces of the interior of a car. The device 1 can incorporate a unique filling system, which prevents foreign material from entering into the unit, including fluids or substances that would deteriorate the performance of the unit or the components of the device I itself. It can utilize an automatic timed shut-off circuit, a fluid level indicator/window and a positioning level indicator. The inside of the device can include an internal anti-foam and anti-splash structure designed to keep electronic components dry and reduce the foaming and turbulence of the fluid being misted. It can also include a metal plate within a plastic embodiment to increase ultrasonic transducer 42 performance.

## Claims

1. A mist-generating machine (1), configured to fit in a vehicle,
the machine (1) comprising:
a misting chamber (41c);
at least one ultrasonic piezoelectric transducer (42) mounted within the misting chamber (41c) of the mist-generating machine (1);
**characterized in that** said machine (1) further comprises:
an adapter (7) configured to enable the mist-generating machine to be in electrical connection with a Low Direct Current Voltage (DCV) power supply provided by a vehicle;
and **in that** ribs (44a-c, 45) are provided within the misting chamber (41c) around the at least one ultrasonic piezoelectric transducer (42), and said ribs (44a-c, 45) are configured to reduce foaming and splashing of the designated fluid covering the at least one ultrasonic piezoelectric transducer (42).

2. The mist-generating machine (1) of Claim 1, further comprising an inlet (4) with a check valve (43) mounted under a cutter (31)the check valve (43) being located between the inlet (4) and the misting chamber (41c), wherein said check valve (43) prevents fluid entering the mist-generating machine (1) by gravity.

3. The mist-generating machine (1) of Claim 1, configured to accept a refill container (28).

4. The mist-generating machine (1) of Claim 1, connectable to a refill container (28), wherein the inlet (4) includes a cutter (31) for cutting open a foil of the refill container (28) as the refill container (28) is locked into place on the mist-generating machine (1).

5. The mist-generating machine (1) of Claim 1, further comprising a trap mechanism (20) that blocks the entry of materials through an outlet (12) of the misting chamber (41c), the trap (20) including two protrusions that extend from the trap (20) and enable the trap (20) to pivot between an up and a down position.

6. The mist-generating machine (1) of Claim 1, further comprising a helix shaped piece (27) mounted within an outlet (12) of the misting chamber (41c).

7. The mist-generating machine (1) of Claim 1, further comprising a visual fluid level indicator, a positioning level indicator, and an automatic timed shut-off circuit.

8. The mist-generating machine (1) of Claim 1, wherein the ribs (45) are configured to have an S-shape.

9. The mist-generating machine (1) of Claim 1, further comprising
a visual code display to indicate a status of treatment; or
a remote visual code display to indicate a status of treatment; or
a readable visual code on a display that indicates the status of treatment.

10. The mist-generating machine (1) of Claim 1, further comprising
a remote display that notifies an operator of a status of treatment; or
a wireless remote display that notifies an operator of a status of treatment; or
a wireless remote display that notifies an operator of a status of treatment and a status of the mist-generating machine (1).

11. The mist-generating machine (1) of Claim 1, further comprising a plurality of blowers that generate airflow through the mist-generating machine (1).

## Patentansprüche

1. Sprühnebel-erzeugendes Gerät (1), welches ausgestaltet ist, in ein Fahrzeug zu passen, worin das Gerät (1) umfasst:
eine Nebel-bildende Kammer (41c);
mindestens einen piezoelektrischen Ultraschall-Transducer (42), der in der Nebel-bildenden Kammer (41c) des Sprühnebel-erzeugenden Geräts (1) vorgesehen ist;
**dadurch gekennzeichnet, dass** das Gerät (1) weiter umfasst:
einen Adapter (7), der ausgestaltet ist, so dass das Sprühnebel-erzeugende Gerät in elektrischer Verbindung mit einer Niederspannungsgleichstromquelle (DCV) steht, die in dem Fahrzeug vorgesehen ist;
und dass Rippen (44a-c, 45) in der Nebel-bildenden Kammer (41c) um den mindestens einen piezoelektrischen Ultraschall-Transducer (42) vorgesehen sind, und dass die Rippen (44a-c, 45) ausgestaltet sind, Schaumbildung und Spritzen des betreffenden Fluids zu reduzieren, das den mindestens einen piezoelektrischen Ultraschall-Transducer (42) bedeckt.

2. Sprühnebel-erzeugendes Gerät (1) nach Anspruch 1, welches weiter umfasst, einen Einlaß (4) mit einem Absperrventil (43), das unter einem Cutter (31) vorgesehen ist, worin das Absperrventil (43) zwischen dem Einlaß (4) und der Nebel-bildenden Kammer (41c) vorgesehen ist, worin das Absperrventil (43) verhindert, dass das Fluid mittels Schwerkraft in das Sprühnebel-erzeugende Gerät (1) gelangt.

3. Sprühnebel-erzeugendes Gerät (1) nach Anspruch 1, das ausgestaltet ist, einen a Nachfüllbehälter (28) aufzunehmen.

4. Sprühnebel-erzeugendes Gerät (1) nach Anspruch 1, welches mit einem Nachfüllbehälter (28) verbunden werden kann, worin der Einlaß (4) einen Cutter (31) zum Aufschneiden einer Folie des Nachfüllbehälters (28) umfasst, wenn der Nachfüllbehälter (28) an Stelle auf dem Sprühnebel-erzeugenden Gerät (1) angebracht ist,.

5. Sprühnebel-erzeugendes Gerät (1) nach Anspruch 1, welche weiter umfasst, einen Fallen-Mechanismus (20), der den Eintritt von Materialien durch den Auslaß (12) der Nebel-bildenden Kammer (41c) verhindert, worin die Falle (20) zwei Vorsprünge aufweist, die sich von der Falle (20) erstrecken und ermöglichen, dass die Falle (20) zwischen einer offenen und geschlossenen Position schwenkt.

6. Sprühnebel-erzeugendes Gerät (1) nach Anspruch 1, welche weiter umfasst, ein Helix-förmiges Stück (27), das in einem Auslaß (12) der Nebel-bildenden Kammer (41c) befestigt ist.

7. Sprühnebel-erzeugendes Gerät (1) nach Anspruch 1, welche weiter umfasst, eine sichtbare Fluidmengen-Anzeige, eine Positionierungshebel-Anzeige, und einen automatischen Zeit-gesteuerten Abschalt-Schaltkreis.

8. Sprühnebel-erzeugendes Gerät (1) nach Anspruch 1, worin die Rippen (45) ausgestaltet sind, eine S-Form aufzuweisen.

9. Sprühnebel-erzeugendes Gerät (1) nach Anspruch 1, welche weiter umfasst
eine Code-Sichtanzeige zum Anzeigen eines Behandlungsstatus; oder
eine Remote-Code-Sichtanzeige zum Anzeigen eines Behandlungsstatus; oder
einen lesbaren sichtbaren Code auf einer Anzeige, die den Status der Behandlung anzeigt.

10. Sprühnebel-erzeugendes Gerät (1) nach Anspruch 1, welche weiter umfasst
eine Remote-Anzeige, die einen Betreiber über den Behandlungsstatus informiert; oder
eine Drahtlos-Remote-Anzeige, die einen Betreiber über den Behandlungsstatus informiert; oder
eine Drahtlos-Remote-Anzeige, die einen Betreiber über den Behandlungsstatus und einen Status des Sprühnebel-erzeugenden Gerät (1) informiert.

11. Sprühnebel-erzeugendes Gerät (1) nach Anspruch 1, welche weiter umfasst, mehrere Lüfter, die einen Luftstrom durch das Sprühnebel-erzeugende Gerät (1) erzeugen.

## Revendications

1. Appareil (1) générateur de brouillard, configuré pour aller dans un véhicule, l'appareil (1) comprenant :
une chambre de brumisation (41c) ;
au moins un transducteur piézoélectrique ultrasonore (42) installé dans la chambre de brumisation (41c) de l'appareil générateur de brouillard (1) ;
**caractérisé en ce que** ledit appareil (1) comprend en outre :
un adaptateur (7) configuré pour permettre l'appareil générateur de brouillard d'être raccordé en connexion électrique avec une alimentation électrique en courant continu basse tension (DCV) assurée par un véhicule ;
et des nervures (44a-c,45) sont pourvues dans la chambre de brumisation (41c) autour d'au moins un transducteur piézoélectrique ultrasonore (42), et lesdites nervures (44a-c, 45) sont configurées pour réduire la formation de mousse et la formation d'éclaboussures dans le fluide désigné recouvrant au moins un transducteur piézoélectrique ultrasonore (42).

2. Appareil (1) générateur de brouillard selon la revendication 1, comprenant en outre une entrée (4) avec un clapet anti-retour (43) installé sous une coupeuse (31), le clapet anti-retour (43) étant situé entre l'entrée (4) et la chambre de brumisation (41c), dans lequel ledit clapet anti-retour (43) empêche la pénétration du fluide dans l'appareil générateur de brouillard (1) par gravité.

3. Appareil (1) générateur de brouillard selon la revendication 1, configuré pour recevoir un récipient de recharge (28).

4. Appareil (1) générateur de brouillard selon la revendication 1, raccordé à un récipient de recharge (28) dans lequel l'entrée (4) comprend une coupeuse (31) pour faire une coupe d'ouverture d'un film du récipient de recharge (28) lorsque le récipient de recharge (28) est verrouillé en place sur l'appareil générateur de brouillard (1).

5. Appareil (1) générateur de brouillard selon la revendication 1, comprenant en outre un mécanisme de piège (20) qui bloque l'entrée de matériaux à travers la sortie (12) de la chambre de brumisation (41c), le piège (20) comprend deux saillies qui s'étendent à partir du piège (20) et qui permettent au piège (20) de pivoter entre une position haute et une position basse.

6. Appareil (1) générateur de brouillard selon la revendication 1, comprenant en outre une pièce (27) en forme d'hélice installée dans la sortie (12) de la chambre de brumisation (41c).

7. Appareil (1) générateur de brouillard selon la revendication 1, comprenant en outre un indicateur visuel de niveau de fluide, un indicateur de niveau de positionnement, et un dispositif d'arrêt automatique temporisé.

8. Appareil (1) générateur de brouillard selon la revendication 1, dans lequel les nervures (45) sont configurées pour être en forme de S.

9. Appareil (1) générateur de brouillard selon la revendication 1, comprenant en outre
un affichage de code visuel pour indiquer l'état du traitement ; ou
un affichage à distance de code visuel pour indiquer l'état du traitement ; ou
un code visuel lisible sur un affichage qui indique l'état du traitement.

10. Appareil (1) générateur de brouillard selon la revendication 1, comprenant en outre
un affichage à distance qui notifie un opérateur d'un état de traitement ; ou
un affichage à distance sans fil qui notifie un opérateur d'un état de traitement ; ou
un affichage à distance sans fil qui notifie un opérateur d'un état de traitement et d'un état de l'appareil générateur de brouillard (1).

11. Appareil (1) générateur de brouillard selon la revendication 1, comprend en outre une pluralité de ventilateurs qui génèrent un flux d'air à travers l'appareil générateur de brouillard (1).
